(19) **Europäisches Patentamt / European Patent Office / Office européen des brevets**

(11) **EP 4 048 779 B1**

(12) **EUROPEAN PATENT SPECIFICATION**

(45) Date of publication and mention
of the grant of the patent:
**05.08.2026 Bulletin 2026/32**

(21) Application number: **20927897.7**

(22) Date of filing: **26.03.2020**

(51) International Patent Classification (IPC):
*C12N 5/00* (2006.01)    *G01N 33/483* (2006.01)
*B81B 1/00* (2006.01)    *G01N 15/10* (2024.01)

(52) Cooperative Patent Classification (CPC):
**G01N 33/48728; G01N 1/38; G01N 15/1031;**
G01N 2015/1006

(86) International application number:
**PCT/US2020/025044**

(87) International publication number:
**WO 2021/194497 (30.09.2021 Gazette 2021/39)**

(54) **CELL PREPARATION WITH A SERIES OF DETECTION DEVICES**

ZELLPRÄPARATION MIT EINER REIHE VON DETEKTIONSVORRICHTUNGEN

PRÉPARATION CELLULAIRE AVEC UNE SÉRIE DE DISPOSITIFS DE DÉTECTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**31.08.2022 Bulletin 2022/35**

(73) Proprietor: **Hewlett-Packard Development
Company, L.P.
Spring TX 77389 (US)**

(72) Inventors:
• **SHKOLNIKOV, Viktor**
**Palo Alto, California 94304 (US)**
• **WARD, Kenneth J.**
**Corvallis, Oregon 97330 (US)**

(74) Representative: **Haseltine Lake Kempner LLP
One Portwall Square
Portwall Lane
Bristol BS1 6BH (GB)**

(56) References cited:
EP-A1- 3 805 754        WO-A1-2017/083391
WO-A1-2018/071731    CN-A- 110 835 596
US-A- 5 376 878          US-A1- 2016 167 051
US-A1- 2017 128 941    US-A1- 2018 016 539
US-A1- 2020 070 167

• **PARHAM GHASSEMI: "Multi-Constriction Microfluidic Sensors for Single-Cell Biophysical Characterization", THESIS, 19 December 2017 (2017-12-19), Blacksburg, Virginia, XP055736826, Retrieved from the Internet <URL:https://vtechworks.lib.vt.edu/bitstream/handle/10919/89947/Ghassemi_P_T_2017.pdf?sequence=1&isAllowed=y> [retrieved on 20201006]**
• **GHASSEMI PARHAM ET AL: "Unique Impedametric Cell Deformability Assay Using a Multi-Constriction Microfluidic Biosensor", 2019 20TH INTERNATIONAL CONFERENCE ON SOLID-STATE SENSORS, ACTUATORS AND MICROSYSTEMS & EUROSENSORS XXXIII (TRANSDUCERS & EUROSENSORS XXXIII), IEEE, 23 June 2019 (2019-06-23), pages 1059 - 1062, XP033599978, DOI: 10.1109/TRANSDUCERS.2019.8808384**
• **YANG DAHOU ET AL: "Biophysical phenotyping of single cells using a differential multiconstriction microfluidic device with self-aligned 3D electrodes", BIOSENSORS AND BIOELECTRONICS, vol. 133, 7 March 2019 (2019-03-07), pages 16 - 23, XP085659100, ISSN: 0956-5663, DOI: 10.1016/J.BIOS.2019.03.002**

EP 4 048 779 B1

**(Cont. next page)**

- **GHASSEMI PARHAM ET AL: "Post-enrichment circulating tumor cell detection and enumeration via deformability impedance cytometry", BIOSENSORS AND BIOELECTRONICS, ELSEVIER SCIENCE LTD, UK, AMSTERDAM , NL, vol. 150, 11 November 2019 (2019-11-11), XP086018344, ISSN: 0956-5663, [retrieved on 20191111], DOI: 10.1016/J.BIOS.2019.111868**

## Description

### BACKGROUND

Microbiology is a field of biology that studies the structure, function, and operation of cells. With an understanding of the structure, function, and operation of cells a variety of chemical reactions and processes can be carried out. For example, individual cells may be used to generate cell lines and to aide in the further understanding of mechanisms of cellular function. US5376878A discloses a multiple-aperture particle counting, sizing and deformability-measuring apparatus. US2018016539A1 discloses disruption and field enabled delivery of compounds and compositions into cells. WO2018071731A1 discloses mechano-node pore sensing. US2020070167A1 discloses processing systems for isolating and enumerating cells or particles. Parham Ghassemi discloses multi-constriction microfluidic sensors for single-cell biophysical characterization in a thesis from Blacksburg, Virginia. Parham Ghassemi et al disclose a unique impedametric cell deformability assay using a multi-constriction microfluidic biosensor in the 20th international conference on solid-state sensors, actuators and microsystems & eurosensors of the IEEE. Yang Dahou et al disclose biophysical phenotyping of single cells using a differential multiconstriction microfluidic device with self-aligned 3d electrodes in biosensors and bioelectronics, vol. 133, pages 16 - 23.

### BRIEF DESCRIPTION OF THE DRAWINGS

The accompanying drawings illustrate various examples of the principles described herein and are part of the specification. The illustrated examples are given merely for illustration, and do not limit the scope of the claims.

Fig. 1 is a block diagram of a cell preparation system with a series of detection devices, according to an example of the principles described herein.

Fig. 2 is a diagram of a cell preparation system with a series of detection devices, according to an example of the principles described herein.

Fig. 3 is a flow chart of a method of cell preparation with a series of detection devices, according to an example of the principles described herein.

Fig. 4 is a flow chart of a method of cell preparation with a series of detection devices, according to an example of the principles described herein.

Fig. 5 is a diagram of a cell preparation system with a series of detection devices, according to an example of the principles described herein.

Fig. 6 is a diagram of a cell preparation system with a series of detection devices, according to an example of the principles described herein.

Fig. 7 is a diagram of a cell preparation system with a series of detection devices, according to an example of the principles described herein.

Fig. 8 is a diagram of a cell preparation system with a series of detection devices, according to an example of the principles described herein.

Fig. 9 is a block diagram of a cell preparation system with a series of detection devices, according to an example of the principles described herein.

Fig. 10 is a block diagram of a cell preparation system with a series of detection devices, according to an example of the principles described herein.

Fig. 11 is a diagram of a cell preparation system with a series of detection devices, according to an example of the principles described herein.

Fig. 12 is a diagram of a cell preparation system with a series of detection devices, according to an example of the principles described herein.

Fig. 13 is a diagram of a cell preparation system with a series of detection devices, according to an example of the principles described herein.

Throughout the drawings, identical reference numbers designate similar, but not necessarily identical, elements. The figures are not necessarily to scale, and the size of some parts may be exaggerated to more clearly illustrate the example shown. Moreover, the drawings provide examples and/or implementations consistent with the description; however, the description is not limited to the examples and/or implementations provided in the drawings.

### DETAILED DESCRIPTION

Microbiology is a field of biology that studies the structure, function, and operation of cells. A greater understanding of the different kinds of cells and their function can lead to certain technological innovations that benefit society in countless ways. For example, from cells, certain biologics such as proteins, insulin, other therapeutic drugs, RNA, and DNA may be synthesized.

Accordingly, refinements to cellular processing may enhance the possible uses and reach of cellular analytics. For example, pure cell lines are used to ensure high fidelity production of biologics as well as for understanding the fundamental mechanisms of cellular function. A pure cell line refers to cells that are derived from a single cell with well-defined properties. Accordingly, to generate such pure cell lines, or biologics from a single cell line, a scientist should be confident that 1) the cell line was derived from a single cell and 2) that the cell has certain desired properties. Determining that a single cell, and the pure cell line derived therefrom, has certain desired properties may increase the efficiency of cellular analytics as it may reduce the number of cells to be cultured as those that do not have the desired property may be distinguished from those that do have the desired property thus reducing the amount of quality control needed for the cultured cells.

As a specific example, a scientist may desire to generate chimeric antigen receptor (CAR) T-cells in order to combat cancerous cells in a patient. CAR T-cell therapy changes a patient's T-cells in a way that attacks the cancerous cells. Accordingly, T-cells are processed and manipulated to form CAR T-cells. Were a starting population to include an unknown quantity of T-cells or to include other types of cells, the quantity and quality of produced CAR T-cells would be reduced and/or unknown, which could affect the efficacy of a session of the CAR T-cell therapy.

Accordingly, by using a starting population for which the characteristics are definitively verified, confidence and reliability in the subsequent operations is promoted. Accordingly, the present specification ensures such reproducibility and confidence by automatically sorting cells based on examination of the cells' multiple electrical and mechanical properties and placing individual cells into individual wells for further propagation or analysis. That is, in the above example, T-cells may be placed in individual wells such that a user may know with certainty what cells and what quantity of cells are present in a starting population such that a deterministic operation may be carried out to generate the CAR T-cells. That is, the present specification reduces the unknowns from a starting population such that a scientist may more accurately carry out subsequent chemical operations.

In other words, as can be imagined, uncertainty regarding the characteristics of a starting population introduces uncertainty and inaccuracy for any operation subsequently carried out on that starting population, whether that operation be analysis to identify a particular relationship, cell generation, and/or biologic generation. The present specification generates a starting population where uncertainty is removed by 1) ensuring single cells are used to generate a pure line and 2) ensuring that the pure line is based off a cell with desired properties. Accordingly, there can be greater confidence in the outcome of any subsequent operation.

While some solutions have been presented, they are inadequate for any number of reasons. For example, in some cases cells may be sorted by fluorescence activated cell sorting (FACS) and magnetically activated cell sorting (MACS), both of which rely on labels to identify the cells, a single cell at a time. However, the presence of such labels may interfere with cellular operation and analysis of cell functionalities. Accordingly, the present specification provides or such sorting and singulation without labeling agents, thus preserving the integrity of any subsequent operations.

Some label-free separation methods exist. Examples include density gradient centrifugation separation and filtration-based (size and stiffness based) cell separation. However, these operations are carried out on large quantities of cells and properties of individual cells are not measured.

Label free singulation of cells may include expensive systems involving video detection integrated with dispensing, or dilution of the cell suspension and portioning the suspension into individual volumes (wells). However, with these methods, a large number of wells may end up without cells and just reagent, while other wells may contain more than one cell. Having wells with one more cell may introduce additional quality control operations which is ineffective and costly.

Accordingly, the present system provides automated, low-cost single-cell sorting and dispensing based on a cell's electrical and mechanical properties. The system includes a microfluidic device with integrated pumps, and a plurality of constrictions with integrated sensors. The sensors are impedance sensing electrodes. Each constriction with a sensor forms an independent detection device, each detection device to measure different aspects of the cell properties. Via a controller, each individual detection device then determines a characteristic of the cell found within the detection device. The controller combines the readings from each detection device and decides whether to eject a cell into a well or to eject the cell to a waste receptacle. Direct combination (Boolean "AND") of the detection device outputs may lead to an exponential increase in the accuracy of obtaining the desired cell.

In one particular example, the system includes a microfluidic chip that includes thermal inkjet (TIJ) dispensing pumps, and a plurality of chambers with a constriction and electrodes, an X-Y stage a control unit to control the stage and the voltages in the microfluidic chip, and a reservoir for cells feeding into the chip. The chambers constrict the cell so that the cell geometry between electrodes is reproducible. In one particular example, a voltage is applied to the electrodes at an appropriate frequency. This generates an electric field. Depending on the electric field and polarizability of the cell membrane, the electric field either penetrates the cell or passes in a thin film of liquid between the cell wall and the constriction. Depending on the elasticity of the cell, which may be a function of the cell microtubule content, membrane content etc., this thin film has a different thickness, and so different impedance. For different cell types, the electric field penetrates the cell membrane at a different frequency. A different field frequency is applied to different chambers, and by comparing the impedance at different chambers, cells can be differentiated on their polarizability, one cell at a time.

Once the cell effective impedance at various frequencies is measured, it is then correlated to both the cell's mechanical properties (deformability during constriction) as well as the membrane electrical properties (membrane capacitance). The controller then either 1) dispenses the cell into an individual well or disposes of the cell and in some cases 2) records the cell properties, which can be further correlated with the cell health (including ability to produce biologics) and other properties analyzed downstream.

The decision to dispense the cell into the well or dispose of the cell is done by consensus of $n$ detection devices. That is, each cell may evaluate whether there is a single cell in the chamber and whether it is of a desired type. Accordingly, if *n* detection devices agree that the cell is of a desired cell, each with a true positive rate, *TP*, and a false positive rate, *FP*, and the frequency of desired cells in the solution is *q*, then the probability, *p*, that the cell is actually a desired cell scales exponentially with the number of independent detectors:

$$p(S|W_1 ... W_n I) = q \left( \frac{TP}{qTP+(1-q)FP} \right)^n$$ . Thus, a plurality of detection devices allows for low-cost high-fidelity cell sorting.

Specifically, the present specification describes a cell preparation system as defined in claim 4.

In some examples, the controller identifies, per detection device and based on output from the sensors, a cell elasticity, a cell size, and a cell effective conductivity and permittivity. The controller may also determine, per detection device, whether an output of a sensor is indicative of a single cell of a desired type.

The sensor is an impedance sensor to measure an impedance within a respective constriction. In some examples, the sensor is disposed within the constriction. In other examples, the sensor is disposed outside the constriction.

In some examples, at least one of a constriction shape and an applied electrical field vary for at least one detection device. In some examples, the cell preparation system includes a database. The database is to map, per detection device, sensor outputs to quantity and types of cells and is indexed based on properties of a respective detection device.

The present specification also describes a method as defined in claim 1.

In one example, based on electrical impedance measures from each detection device, the method includes determining whether to eject the cell onto a target surface or to dispose of the cell to a waste receptacle. In some examples, properties for each cell are recorded.

The present specification also describes a cell preparation system. The cell preparation system includes a signal generator to generate electrical fields to apply to multiple detection devices and at least one fluidic channel to transport cells in single file past multiple detection devices. The cell preparation system also includes a series of detection devices per fluidic channel, each detection device including: a constriction to deform a cell found therein and at least one electrode pair to measure an electrical property within the constriction. The cell preparation system also includes sense circuitry to determine an impedance within each detection device and an ejector to eject cells from the fluidic channel. The cell preparation system also includes a controller to aggregate outputs form the multiple electrode pairs to: verify a single file transport of cells through the system; identify a cell that passes through the fluidic channel; and determining whether to eject the cell onto a target surface or to dispose of the cell to a waste receptacle.

In some examples, the system includes a filter device per detection device to generate an electrical field specific to a respective detection device. The filter device may be selected from the group consisting of a band-pass filter and a lock-in amplifier.

In one example, the sense circuitry includes an isolation component to isolate output from a particular detection device. The isolation component may be selected from the group consisting of a band-pass filter and a switch. The sense circuitry may also include a current meter.

In summary, using such a cell preparation system 1) provides highly accurate cell separation and identification; 2) is low cost; 3) provides for the rapid generation of many singulated cells; 4) avoids additional labelling reagents; 5) provides an integrated design; 6) exhibits a low probability of empty wells; and 7) reduces quality control measures. However, the devices disclosed herein may address other matters and deficiencies in a number of technical areas.

As used in the present specification and in the appended claims, the term "effective conductivity and permittivity" refers to a cells ability to conduct electricity and how a cell polarizes by imposition of an electrical field.

Still further, as used in the present specification and in the appended claims "applying an electrical field" refers to controlling a voltage or controlling a current and measuring the other. For example, an electrical field may be applied by controlling a voltage and measuring a current. In another example, applying an electrical field includes controlling a current and measuring a voltage.

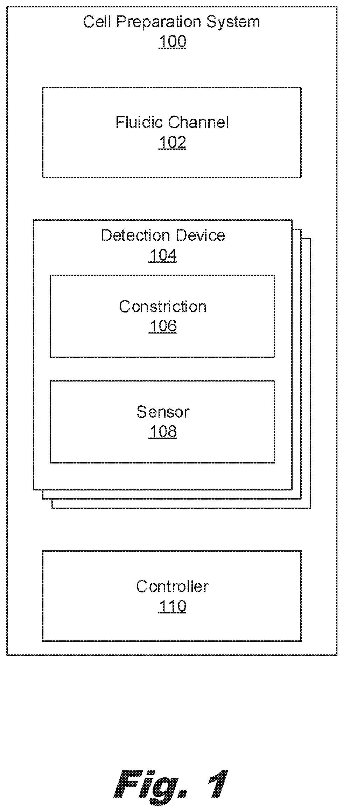

Turning now to the figures, Fig. 1 is a block diagram of a cell preparation system (100) with a series of detection devices (104), according to an example of the principles described herein. In some examples, the cell preparation system (100) is part of a lab-on-a-chip device. A lab-on-a-chip device combines several laboratory functions on a single integrated circuit which may be disposed on a silicon wafer. Such lab-on-a-chip devices may be a few square millimeters to a few square centimeters, and may provide efficient small-scale functionality. In other words, the components, i.e., fluidic channels (102) and detection devices (104) may be microfluidic structures. A microfluidic structure is a structure of sufficiently small size (e.g., of nanometer sized scale, micrometer sized scale, millimeter sized scale, etc.) to facilitate conveyance of small volumes of fluid (e.g., picoliter scale, nanoliter scale, microliter scale, milliliter scale, etc.).

To carry out such cell preparation, the cell preparation system (100) includes a variety of sub-components. Specifically, the cell preparation system (100) includes a fluidic channel (102) to transport cells in single file past multiple detection devices (104). Accordingly, the fluidic channel (102) may be a microfluidic structure coupled at one end to a cell reservoir and directing cells single-file towards the first detection device (104). Following passage by the multiple detection devices (104), the fluidic channel (102) may direct the cells to another destination, such as an ejection chamber to be expelled onto a target surface such as a well of a titration plate or to be expelled to a waste container.

The cell preparation system (100) also includes a series of detection devices (104). As described above, each detection device (104) includes components that aid in accurately and reliably 1) indicating whether cells are transported single-file and 2) identifying particular cells. That is, each detection device (104) in the series outputs data indicating whether a single cell passed through and that is used to identify the cell that passed through. The controller (100) considers similar information for each of the detection devices (104) and determines from the aggregated information whether a single cell passed by and what the type of cell was. The cell preparation system (100) may include any number of multiple detection devices (104). The greater the number of detection devices (104), the higher the accuracy of the cell preparation system (100).

Each detection device (104) includes multiple components. For example, each detection device (104) includes a constriction (106) to deform the cell. Specifically, the constriction (106) may have a cross-sectional size smaller than the cell so as to deform the cell. Accordingly, as noted above, the constriction (106) may be a microfluidic structure. As will be described below, the degree to which the cells deform as they enter the constriction (106) may be used to identify the particular cell. For example, some cells deform more than others and will more readily deform within the constriction (106). The degree of deformation may therefore be used to identify and classify the cell. The degree of deformation may be determined by the sensors (108) and the controller (110).

The detection device (104) also includes a sensor (108) to measure a state within the constriction (106). The sensor (108) is an impedance sensor to measure an electrical conductivity within a respective constriction (106). Such an impedance sensor includes at least one pair of electrodes. These electrodes detect a level of conductivity within the constriction (106). As the contents within the constriction change (106), so does the electrical impedance within the constriction (106). For example, the impedance within the constriction (106) may be different when a cell is present in the constriction (106), as compared to when a cell is not in the constriction (106). Moreover, different types of cells change the impedance by different amounts. Accordingly, the impedance sensors (106) can measure conductivity and an impedance may be determined therefrom. This impedance value can be correlated to determine 1) whether one or multiple cells are found in a constriction (106) and 2) what are the properties of the cell(s) found in the constriction (106).

The cell preparation system (100) also includes a controller (110). The controller (110), which includes hardware components such as a processor and memory, analyzes outputs from multiple sensors (108) to verify a single file transport of cells through the cell preparation system (100) and identifies cells that pass through the fluidic channel (102). That is, as described above, based on the output of not one, but multiple detection devices (104), the controller (110) can determine not only whether cells are being transported in single-file fashion through the fluid channel (102), but can also determine a type of cell, that is its identity. By combining the output of multiple detection devices (104), greater accuracy and reproducibility results. That is, rather than relying on a single detection device (104), which for one reason or another may return skewed results, the controller (110) of the present system relies on output of multiple detection devices (104). The same output from the different detection devices (104) may more assuredly indicate a particular single cell.

Note that in this example, each detection device (110) may apply different electrical fields such that the cell preparation system (100) is not just replicating a detection, but is detecting based on different parameters. That is, a cell is exposed to different experimentational conditions, and when each of the detection devices (104) identifies, based on its specific experimentational conditions, that the cell is of a particular type, the controller (110) may output with a degree of confidence that the cell is of that particular type.

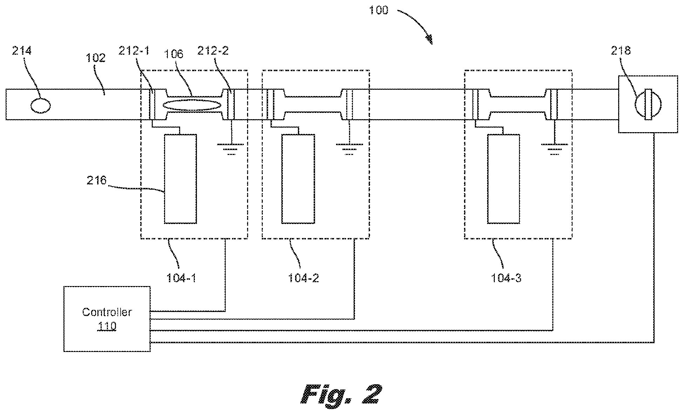

Fig. 2 is a diagram of a cell preparation system (100) with a series of detection devices (104), according to an example of the principles described herein. Fig. 2 clearly describes the various detection devices (104). For simplicity, in Fig. 2 three detection devices (104-1, 104-2, 104-3) are depicted. However, the cell preparation system (100) may include any number of multiple detection devices (104). As described above, the cell preparation system (100) includes a fluidic channel (102) through which a cell (214) passes by the detection devices (104).

As described above, each detection device (104) includes a constriction (106). For simplicity, a single constriction (106) is indicated with a reference number. However, each detection device (106) includes a constriction (106). Different cells (214) may deform differently when forced through a constriction (106). That is, some cells (214) are more elastic and may more readily deform. The elasticity of a cell (214) may be used to identify and classify a cell (214) and the sensors (Fig. 1, 108) as described above may detect the deformation of a cell (214) passing through the constriction (106).

Fig. 2 also clearly depicts the sensor (Fig. 1, 108) which includes two electrodes (212-1, 212-2) placed on either side of the constriction (106). In the example depicted in Fig. 2, the sensor (Fig. 1, 108) is disposed outside the constriction (106). Doing so enables a uniform electrical field inside the constriction (106). As with the constriction (106), Fig. 2 references just two electrodes (212-1, 212-2) with a reference number, however the other detection devices (104) may include similar structures.

One electrode (212-1) may be coupled to a source (216) while a second electrode (212-2) is coupled to ground. Between these electrodes (216-1, 216-2) a current may be controlled and a voltage measured. In another example, a voltage is controlled and a current measured. Using both these values, the impedance between the electrodes (212), i.e., within the constriction (106) is determined.

The presence of a cell (214) within a constriction (106) will effectively change the electrical conductivity within the constriction (106). Moreover, different types of cells (214) affect the electrical conductivity to different degrees. That is, some cells (214) may have a larger impedance and increase the effective impedance within the constriction (106) to a different degree as compared to other cells (214) that may have a smaller impedance. Accordingly, the controller (110) may rely on a mapping between impedance values and cell (214) types to aide in identifying the cell. Part of the difference in impedance may be attributable to a cell (214) size. Accordingly, the cell (214) size may be determined based on a determined impedance value.

Even further, a particular cell (214) when exposed to different electrical fields may affect the conductivity to differing degrees. For example, a particular cell (214) when exposed to a lower voltage may be insulative, but when exposed to a higher voltage may be conductive. Accordingly, by probing the cell (214) at different voltages/currents the membrane properties, i.e., conductivity and permittivity of the cell, may be determined. Specifically, each of the different sensors (Fig. 1, 108) may apply a different electric field. As a cell (214) passes through the different electrical fields, it may be determined at what point the cell (214) changes from conductive to insulative, or vice versa. Via a mapping, the controller (110) may determine how the transition point indicates the type of the cell (214).

Even further, a quantity of cells (214) in a constriction (106) may affect the conductivity. That is, more cells (214) may have a larger effect on impedance than a single cell. Accordingly, the controller (110) may map measured impedances with a quantity of cells (214).

In addition to using information from the cell (214) to identify the cell (214), information relating to the impedance of the surrounding fluid may also be used to identify the cell (214). That is, as described above, different cells (214) have different elasticities. Depending on the elasticity of the cell (214), it may deform to different degrees. The more a cell (214) deforms, the less carrier fluid will be present in the constriction (106). Whereas a more rigid cell (214), which deforms less, will result in more carrier fluid present in the constriction (106). The amount of fluid, and in particular the amount of fluid between the cell (214) walls and the walls of the constriction (106), results in different impedance values. Accordingly, the impedance within a constriction (106) can be correlated to a thickness of the film between the cell (214) and the constriction (106) wall. This thickness can then be mapped to a flexibility of the cell (214), and such flexibility used to classify the cell (214).

Note that in these examples, the impedance in the region of the constriction (106) can be used to determine different characteristics of the cell (214) such as cell effective permittivity and conductivity, cell elasticity, and cell count. To determine these values, impedance is measured as a function of constriction geometry and frequency and magnitude of the electric field.

As a specific example, cell effective permittivity and conductivity, cell elasticity, and cell size may be determined based on a correlation between an electrical signal such as impedance and the cell properties. For example, a mammalian cell (214) may have an elasticity of between 1 - 10 kilopascals (kPa). Cell elasticity may be measured via force microscopy. For example, a cantilever may be pressed with a known pressure into a cell while its under microscopic observation. The slope of the line of the applied pressure vs. deformation (strain) is the elastic modulus.

A mammalian cell (214) may have an electrical conductivity of 0.1 - 2 Siemens per meter (S/m) and a permittivity of 2 - 60 x vacuum permittivity where vacuum permittivity is $8.85 \times 10^{-12}$ Farad per meter (F/m). To determine electrical conductivity and permittivity, the cell's (214) motion is observed in an AC electric field at various frequencies. The motion of the cell depends on the electric force applied to the cell (214), which is in turn related to its effective conductivity and permittivity.

A mammalian cell (214) may have a cell size of between 10 and 15 micrometers. However, mammalian cells may be as small as 3 micrometers and as large as 100 micrometers. Cell sizes may be determined by microscopic observation.

Accordingly, a mammalian cell (214) is introduced into the cell preparation system (100), and passes by multiple detection devices (104). However, at the time of introduction, it may be unknown that the cell (214) is a mammalian cell.

As the cell passes through the cell preparation system (100), it passes by a series of detection devices (104). Each detection device (104) includes a constriction (106) and a sensor (Fig. 1, 108) such as an impedance sensor. The impedance sensor (Fig. 1, 108) operates to detect an impedance and/or a conductivity within the volume of the constriction (106). Specifically, the sensor (Fig. 1, 108) includes at least one pair of electrodes (212) spaced apart from one another by a gap. These electrodes (212) detect a level of impedance within the constriction (106). That is, incoming cells to a constriction (106), and the solution in which they are contained, have a predetermined electrical conductivity. Any change to the contents within the constriction (106), will effectively change the electrical conductivity and impedance within the constriction (106). Specifically, as the cells enter and deform within the constriction (106), carrier fluid is displaced and the impedance within the constriction (106) changes as a result.

Accordingly, as a cell passes through different detection devices (104), the corresponding electrodes (212) and controller (110) determine an impedance within the constriction (106). Specifically, a voltage is applied to the electrodes (212) by the controller (110). The controller (110) determines a ratio of 1) the voltage applied to the electrodes (212) to 2) the current flowing through the electrodes (212) and determines an impedance therefrom.

These electrical impedance measurements are then correlated to the different characteristics, i.e., cell elasticity, electrical conductivity, and electrical permittivity. That is, there may be a correlation table that maps output impedances to known values of each characteristic. Accordingly, once an output impedance is known, the controller (100) may identify that output impedance in the correlation table and a corresponding elasticity, electrical conductivity, electrical permittivity, and/or cell size may be identified.

As described above, each characteristic, i.e., conductivity, permittivity, size, and elasticity, may be determined by a separate detection device (104). For example, a controller (110) may map an output from a first detection device (104-1) to a cell elasticity and may map an output from a second detection device (104-2) to a cell conductivity.

In the case where a mammalian cell is introduced, output impedances may be determined by the sensor (Fig. 1, 108) and using a database or other correlation table which maps this output impedance to the aforementioned parameters, the controller (110) may identify the cell. That is, given that a mammalian cell has been introduced into the system, the elasticity, electrical conductivity, permittivity and size of the cell would fall within the ranges noted above. Accordingly, the controller (110) may verify that a mammalian cell has passed through the cell preparation system (100) by measuring electrical impedances and correlating those electrical impedances to known values of certain characteristics. Those certain characteristics can then be mapped to a particular cell. More precise measures of each value, which may be determined from the correlation table, may allow for a more refined cell identification, such as what type of mammalian cell passed through.

Accordingly, once either of these characteristics (i.e., cell effective permittivity and conductivity, cell elasticity, and cell count) is determined, that variable may be marked as known, and the other characteristic may be determined. Accordingly, by probing the cell (214) at different voltages/currents, and frequencies the controller (110) can determine either the dimensions of the film around the cell (214), or the properties of the cell (214) itself. Once the properties of the film around the cell (214) are determined, this is removed as an unknown and other properties of the cell (214), such as the properties of the cell (214) itself can be determined in downstream detection devices (104).

Accordingly, the controller (110) identifies, per detection device (104) and based on an output from the sensors (Fig. 1, 108), a cell elasticity, a cell size, and a cell effective conductivity and permittivity.

The controller (110) also determines, per detection device (104), whether an output of a sensor (Fig. 1, 108) is indicative of a single cell (214) of a desired type. That is, as described above, each detection device (104) may test for one of the aforementioned characteristics and the combined results may lead the controller (110) to determine that the cell is of a particular type. For example, through a first detection device (104-1) it may be determined that there is a single cell (214) as opposed to multiple cells (214) present in a constriction (106). In a second detection device (104-2), a certain thickness of film surrounding the cell (214) is determined such that an elasticity value for the cell (214) is determined. In subsequent detection devices (104), knowing the elasticity and determined film thickness, different electrical fields may be applied to determine at which point the cell transitions from insulative to conductive or vice-versa, thus providing additional information related to the type of cell (214). Thus, the series of detection devices (104) may determine different characteristics of the cell (214) such that it may be determined whether the cells (214) are serially transported along the fluidic channel (102) and what types of cells (214) are transported.

Based on the output of the different detection devices (104), the controller (110) may take a variety of actions. For example, the cell preparation system (100) may include an ejector (218) that expels the cell (214) onto a target surface such as a well of a titration plate. That is, if the controller (110) determines that there is a single cell (214) of a desired type present, the controller (110) may activate the ejector (218) to expel the cell (214). By comparison, if the controller (110) determines that there is not a single cell (214) of the desired type present, the controller (110) dispose of the cell (214). In one particular example, the controller (110) may activate the ejector (218), but the controller (110) or a different processor may move the target surface such that the undesirable cell (214) is ejected onto a different surface, such as a waste receptacle.

The ejector (218) may include a firing resistor or other thermal device, a piezoelectric element, or other mechanism for ejecting fluid from the firing chamber. For example, the ejector (218) may be a firing resistor. The firing resistor heats up in response to an applied voltage. As the firing resistor heats up, a portion of the fluid in the firing chamber vaporizes to form a bubble. This bubble pushes the cell (214) out the opening and onto a surface such as a micro-well plate. As the vaporized fluid bubble collapses, a vacuum pressure along with capillary force within the firing chamber draws another cell (214) into the firing chamber from the fluidic channel (102), and the process repeats. In this example, the ejector (218) may be a thermal inkjet ejector (218).

In another example, the ejector (218) may be a piezoelectric device. As a voltage is applied, the piezoelectric device changes shape which generates a pressure pulse in the firing chamber that pushes a cell (214) out the opening. In this example, the ejector (218) may be a piezoelectric inkjet ejector (218).

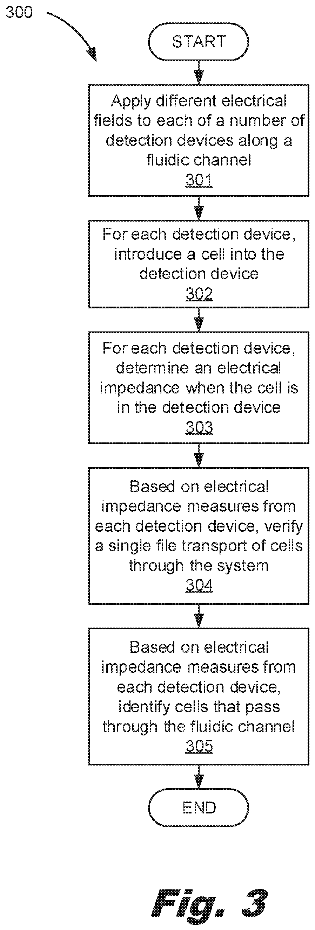

Fig. 3 is a flow chart of a method (300) of cell (Fig. 2, 214) preparation with a series of detection devices (Fig. 1, 104), according to an example of the principles described herein. That is, the cell preparation system (Fig. 1, 100) ensures that a single instance of a particular cell (Fig. 2, 214) is ejected from the cell preparation system (Fig. 1, 100). Doing so enhances reproducibility of subsequent operations, reduces variance within starting samples, and reduces quality control measures as a scientist may have confidence that a particular well includes just a single instance of a target cell (Fig. 2, 214).

According to the method (300), different electrical fields are applied (block 301) to each of multiple detection devices (Fig. 1, 104) along a fluidic channel (Fig. 1, 102). That is, as a cell (Fig. 2, 214) travels along the fluidic channel (Fig. 1, 102) past different detection devices (Fig. 1, 104) it is exposed to different controlled voltages, which different voltages are used to identify different attributes of the cell (Fig. 2, 214) or are used to verify the attributes under different conditions. For example, to determine an effective conductivity and permittivity of a cell (Fig. 2, 214), the cell (Fig. 2, 214) may be exposed to voltages of increasing or decreasing strength to identify a transition point for the cell (Fig. 2, 214) between appearing conductive and insulative. In another example, the different voltage values may be used to verify a property of the cell (Fig. 2, 214) albeit at different characteristics. That is different electrical fields may be generated at different detection devices (Fig. 1, 104), each of which may be used to verify the type of the cell (Fig. 2, 214) passing thereby.

Then, for each detection device (Fig. 1, 104), a cell (Fig. 2, 214) is introduced (block 302) into a constriction (Fig. 1, 106) of the detection device (Fig. 1, 106). In some examples, the quantity of cells (Fig. 2, 214) are serially passed to each detection device (Fig. 1, 104). That is, each cell (Fig. 2, 214) within the sample may be received one at a time. The constriction (Fig. 1, 106), by having a cross-sectional area size on the order of the cell diameter, gates introduction of one cell (Fig. 2, 214) at a time into a space between the electrodes (Fig. 2, 212).

Also, for each detection device (Fig. 1, 104) an electrical impedance is determined (block 303) when the cell (Fig. 2, 214) is in the constriction (Fig. 1, 106). That is, the electrodes (Fig. 2, 212) control either a current or voltage and measure the corresponding electrical value such that impedance can be determined.

Based on the electrical impedance measures from each detection device (Fig. 1, 104), a single file transport of cells (Fig. 2, 214) through the cell preparation system (Fig. 1, 100) is verified (block 304). This may be done from measurements taken by a single detection device (Fig. 1, 104) or by multiple detection devices (Fig.

1, 104).

Also based on the electrical impedance measures from each detection device (Fig. 1, 104), cells (Fig. 2, 214) that pass through the fluidic channel (Fig. 1, 102) are identified (block 305). For example, different detection devices (Fig. 1, 104) may apply an electrical field, either the same or different, to determine a thickness of a thin film surrounding the cell (Fig. 2, 214). Based on the measured impedance values from the detection devices (Fig. 1, 104) an elasticity of the cell (Fig. 2, 214) may be determined.

Still further, other detection devices (Fig. 1, 104), knowing the elasticity of the cell (Fig. 2, 214) may apply an electrical field, either the same or different, to determine a transition point between when the cell (Fig. 2, 204) switches from being conductive to insulative, or vice-versa.

While an example is provided of first determining a thickness of the thin film then determining effective conductivity and permittivity, in some examples, these operations may be reversed. That is, there may be multiple variables of the cell (Fig. 2 214) that may be determined based on a measured impedance and the present cell preparation system (Fig. 1, 100) and the method (300) allows for the sequential determination of each of those variables by applying different electrical fields to the cell in different detection devices (Fig. 1, 104).

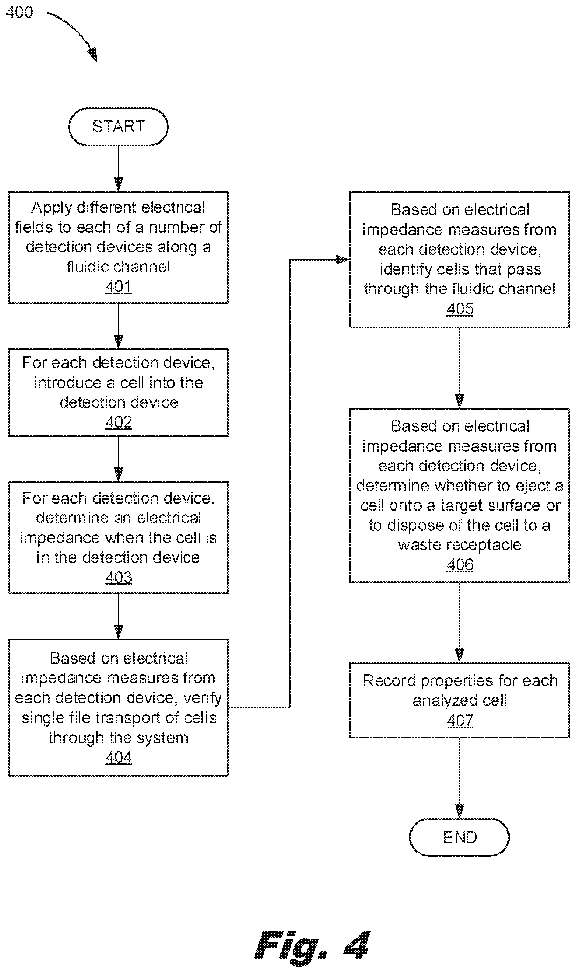

Fig. 4 is a flow chart of a method (400) of cell (Fig. 2, 214) preparation with a series of detection devices (Fig. 1, 104), according to an example of the principles described herein. According to the method (400), different electrical fields are applied (block 401) to each of multiple detection devices (Fig. 1, 104) along a fluidic channel (Fig. 1, 102). This may be performed as described above in connection with Fig. 3.

Moreover, for each detection device (Fig. 1, 104), a cell (Fig. 2, 214) is introduced (block 402) into the detection device (Fig. 1, 104) and an electrical impedance is determined (block 403) when the cell (Fig. 2, 214) is in the detection device (Fig. 1, 102). These operations may be performed as described above in connection with Fig. 3.

Based on electrical impedance measures from each detection device (Fig. 1, 104), single file transport of cells (Fig. 2, 214) through the system is verified (block 404) and cells (Fig. 2, 214) that pass through the fluidic channel (Fig. 1, 102) are identified (block 405). These operations may be performed as described above in connection with Fig. 3.

Based on electrical impedance measures from each detection device (Fig. 1, 104), it is determined (block 406) whether to eject the cell (Fig. 2, 214) onto a target surface or to dispose of the cell (Fig. 2, 214) to a waste receptacle. That is, the controller (Fig. 1, 110) may activate the ejector (Fig. 2, 218) to eject the cell (Fig. 2, 214) into a well plate when it is a single cell with impedance measurements from each detection device (Fig. 1, 104) that verify it is a target cell (Fig. 2, 214). By comparison, when the cell (Fig. 2, 214) is not a target cell (Fig. 2, 214), it may be disposed of. The path to the waste receptacle may be through the ejector (Fig. 2, 218) for example onto a different location on a target surface, or onto an entirely different surface. In other examples, the waste receptacle is on the fluidic die itself.

The method (400) may include recording (block 407) properties for each cell (Fig. 2, 214). That is, regardless of whether the cell is a target cell (Fig. 2, 214) or not, measured impedance values and/or their respective cell (Fig. 2, 214) properties may be communicated to the controller (Fig. 1, 110) which may use the information in any variety of ways. For example, the controller (Fig. 1, 110) may pass the recorded (block 407) properties to a device that carries out any downstream operation such that the results of those downstream operations may be correlated to the recorded (block 407) properties of the cell for analytic, research, or qualifying purposes. That is, additional insight into the outcome/results of a subsequent operation may be had if unique properties for the cell (Fig. 2, 214) exist, these unique properties having been recorded (block 407).

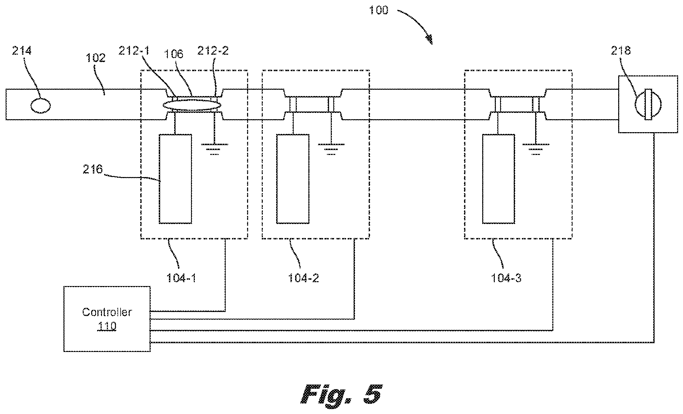

Fig. 5 is a diagram of a cell preparation system (100) with a series of detection devices (104), according to an example of the principles described herein. Fig. 5 depicts various components previously described including the fluidic channel (102), detection devices (104-1, 104-2, 104-3), constriction (106), controller (110), electrodes (212-1, 212-2), source (216), and ejector (218) as well as the cell (214) that is transported through the cell preparation system (100). However, in this example, the sensor (Fig. 1, 108) is disposed within the constriction (106). More specifically, the electrodes (212-1, 212-2) that make up an impedance-type sensor (Fig. 1, 108) are disposed in the constriction (106). Doing so enables a higher density field across the cell (214) and/or allows for a lower voltage to be applied within the constriction (106).

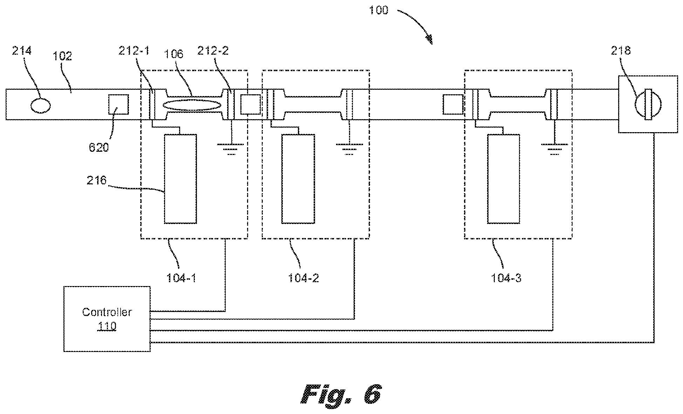

Fig. 6 is a diagram of a cell preparation system (100) with a series of detection devices (104), according to an example of the principles described herein. Fig. 6 depicts various components previously described, including the fluidic channel (102), detection devices (104-1, 104-2, 104-3), constriction (106), controller (110), electrodes (212-1, 212-2), source (216), and ejector (218) as well as the cell (214) that is transported through the cell preparation system (100).

In the example depicted in Fig. 6, the cell preparation system (100) includes a number of pumps (620) to aid in the fluid movement through the fluidic channel (102). In some examples, the pumps (620) may be integrated pumps, meaning the pumps (620) are integrated into a wall of the fluidic channel (102). In some examples, the pumps (620) may be inertial pumps which refers to pumps (620) which are in an asymmetric position within the fluidic channel (102). The asymmetric positioning within the channel (102) facilitates an asymmetric response of the fluid to the pumps (620). The asymmetric response results in fluid displacement when the pumps (620) are actuated. In some examples, a pump (620) may be a thermal inkjet resistor, or a piezo-drive membrane or any other displacement device. While Fig. 6 depicts a pump (620) per detection device (104), the pumps (620) may be disposed upstream of the detection devices (104). In this example, the single pump (620) may direct fluid to all the detection devices (104) found in a cell preparation system (100).

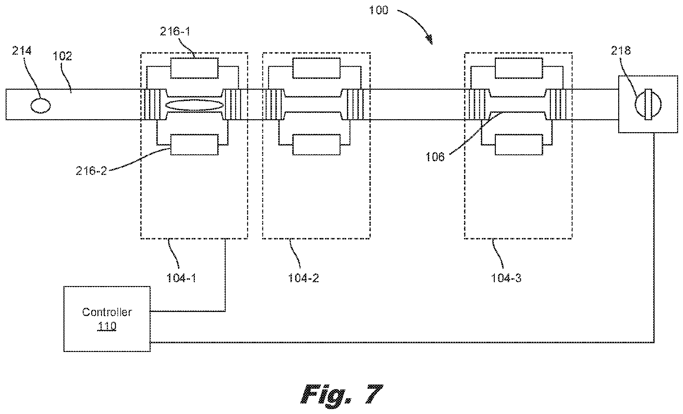

Fig. 7 is a diagram of a cell preparation system (100) with a series of detection devices (104), according to an example of the principles described herein. Fig. 7 depicts various components previously described including the fluidic channel (102), detection devices (104-1, 104-2, 104-3), constriction (106), controller (110), electrodes (212-1, 212-2), source (216), and ejector (218) as well as the cell (214) that is transported through the cell preparation system (100).

In the example depicted in Fig. 7, rather than including a single electrode (212) on either side of the constriction (106), the cell preparation system (100) may include a pair of electrodes (212) on either side of the constriction (106). In this example, voltage may be controlled by one source (216-1) which is coupled to a pair of electrodes and current is measured by a source (216-2) which is coupled to another pair of electrodes (216). From these, an impedance within the constriction (106) may be determined. Such 4-electrode sensing may provide a better signal-to-noise ratio, thus increasing the resolution of the impedance measurements.

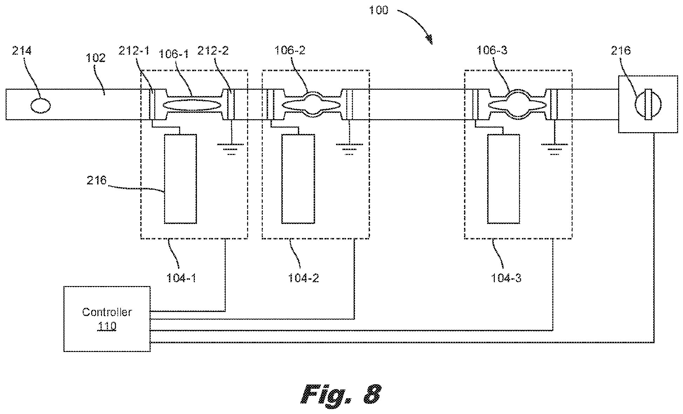

Fig. 8 is a diagram of a cell preparation system (100) with a series of detection devices (104), according to an example of the principles described herein. Fig. 8 depicts various components previously described including the fluidic channel (102), detection devices (104-1, 104-2, 104-3), constriction (106), controller (110), electrodes (212-1, 212-2), source (216), and ejector (218) as well as the cell (214) that is transported through the cell preparation system (100).

In the example depicted in Fig. 8, the cell preparation system (100) includes constrictions (106) of different sizes and/or shapes. That is, at least one of a constriction (106) shape and an applied electrical field vary for at least one detection device (104). That is, a combination of different constriction (106) shapes and different electrical fields may be combined in the cell preparation system (100). For example, a first constriction (106-1) may have a first shape, a second constriction (106-2) may have a second shape, and a third constriction (106-3) may have a third shape.

The different constriction (106) shapes may provide further data regarding the elasticity of the cell (Fig. 2, 214) such that a more accurate classification of the cell (Fig. 2, 214) based on its elastic properties may be made. For example, a more deformable cell (Fig. 2, 214) may better fill the constriction (106), decreasing the liquid film layer, and increasing the impedance. On the other hand, a less deformable cell (Fig. 2, 214) will fill the constriction (106) to a lesser extent, and so lead to a decrease in impedance. Performing such measurements in a variety of constrictions (106) of different shapes and/or sizes provides additional data points by which a cell (Fig. 2, 214) elasticity can be determined.

As depicted in Figs. 2, 5, 6, 7, and 8, the cell preparation system (100) may include a variety of modifications so as to prepare and analyze cells (Fig. 2, 214) in any number of ways. Thus, the present specification provides for a customizable cell preparation system (100) that may be tailored to a particular situation.

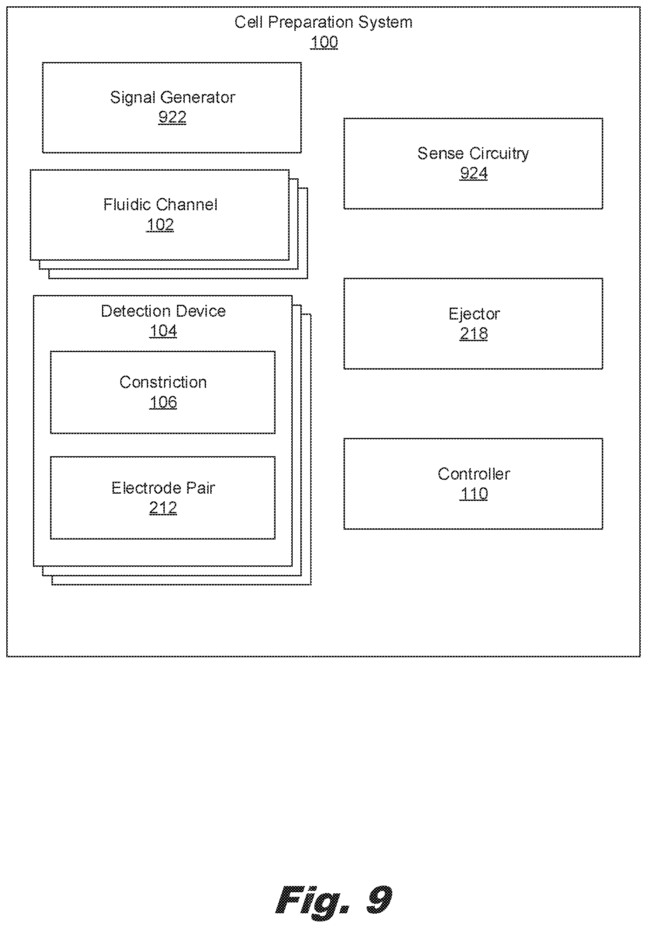

Fig. 9 is a block diagram of a cell preparation system (100) with a series of detection devices (104), according to an example of the principles described herein. In this example, the cell preparation system (100) includes a signal generator (922) to generate electrical fields to apply to multiple detection devices (104). Specifically, the signal generator (922) may control application of a voltage or a current to the different detection devices (104). As described above, the electrical field applied to a particular detection device (104) may be different than the electrical fields applied to other detection devices (104).

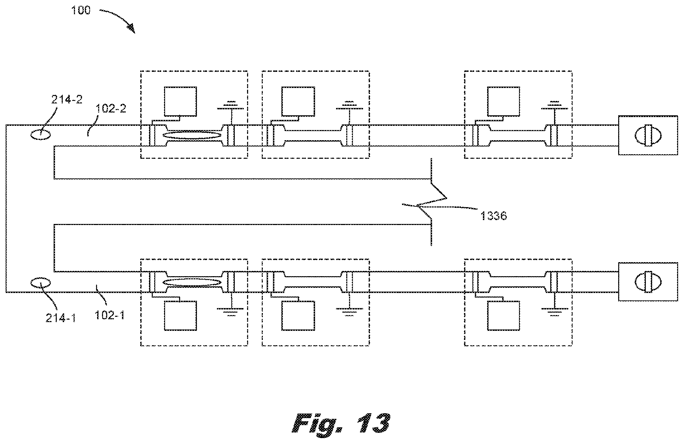

The cell preparation system (100) also includes at least one fluidic channel (102) to transport cells (Fig. 2, 214) in single file past multiple detection devices (104). That is, as has been described in previous examples, the cell preparation system (100) may include a single fluidic channel (102). However, in other examples such as that depicted in Fig. 13, the cell preparation system (100) may include multiple fluidic channels (102) to increase the throughput of the cell preparation system (100).

In this example, as in others, the cell preparation system (100) includes a series of detection devices (104) per fluidic channel (102), each detection device (104) including a constriction (106) to deform a cell (Fig. 2, 214) found therein. As described above, each detection device (104) also includes a sensor (Fig. 1, 108), which in this example includes at least one electrode (212) pair, to measure an electrical property within the constriction (106). That is, when a voltage is controlled, the electrode pair (212) measures a current and when a current is controlled, the electrode pair (212) measures a voltage.

In some examples, the cell preparation system (100) includes sense circuitry (924) to determine an impedance within each detection device (104). That is, the sense circuitry (924) may sense the measured voltage/current from the electrodes (212). The sense circuitry (924) may include an isolation component to isolate output from a particular detection device (104), wherein the isolation component is selected from the group consisting of a band-pass filter and a switch. The sense circuitry (924) also includes a current meter.

The cell preparation system (100) may include the ejector (218) to eject cells (Fig. 2, 214) from the fluidic channel (102). The cell preparation system (100) also includes a controller (110). As described above, the controller (110) aggregates outputs from multiple electrode pairs (212) to 1) verify a single file transport of cells (Fig. 2, 214) through the cell preparation system (100); 2) identify a cell (Fig. 2, 214) that passes through the fluidic channel (Fig. 1, 102); and 3) determine whether to eject the cell (Fig. 2, 214) onto a target surface or to dispose of the cell (Fig. 2, 214) to a waste receptacle.

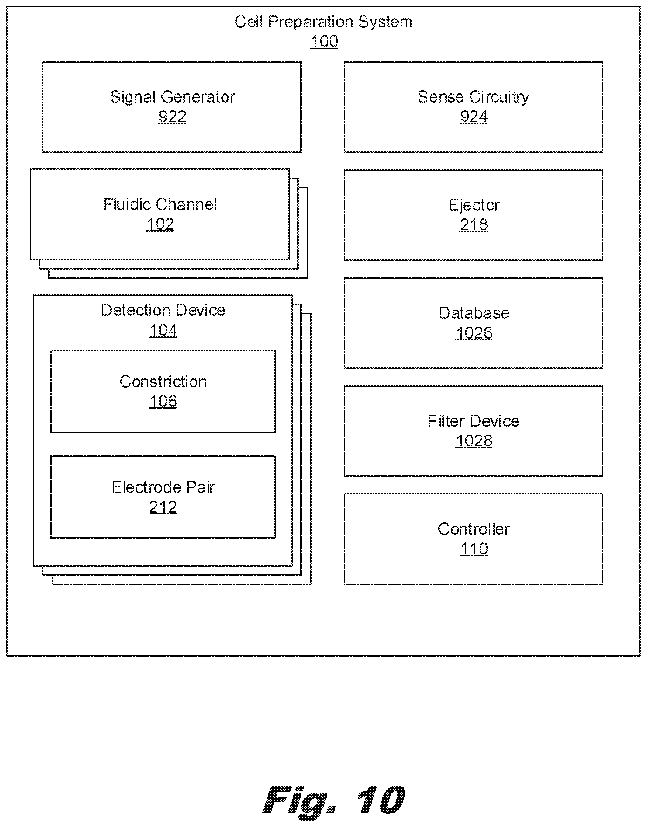

Fig. 10 is a block diagram of a cell preparation system (100) with a series of detection devices (104), according to an example of the principles described herein. In the example depicted in Fig. 10, the cell preparation system (100) includes the signal generator (922), at least one fluidic channel (102), detection device(s) (104) with their constrictions (106) and electrode pairs (212), the sense circuitry (924), ejector (218), and controller (110).

In this example, the cell preparation system (100) includes additional components. For example, the cell preparation system (100) includes a database (1026) to map, per detection device (104), sensor (Fig. 1, 108) outputs to quantity and types of cells (Fig. 2, 214) and that is indexed based on properties of a respective detection device (104). That is, as described above, state measurements, such as impedance measurements, within the constriction (106) are measured. However, these measurements themselves do not identify the cell (Fig. 2, 214). Accordingly, the cell preparation system (100) may include a database (1026) that is remote from the fluidic die. The controller (110) references this database (1026) in determining the cell type. For example, based on information relating to the parameters of the detection, i.e., the impedance measurements, shape/size of the constriction, applied electrical field, etc., the controller (110) may determine properties such as an effective conductivity and permittivity, thin film thickness, quantity of cells, etc. of what is inside the constriction (106) such that a proper identification of the cell (Fig. 2, 214) and whether or not there is a single cell (Fig. 2, 214) may be made.

In this example, the cell preparation system (100) includes a filter device (1028) per detection device (104) to generate an electrical field specific to a respective detection device (104). That is, the signal generator (922) may generate any number of signals, for example as a step function. The filter device (1028) may gate a subset of the generated signals such that just signals having a particular characteristic are passed to a respective detection device (104). In this fashion, different electrical fields, i.e., those allowed by the filter device (1028), may be applied at the different detection devices (104). The filter device (1028) may be selected form the group consisting of a band-pass filter and a lock-in amplifier.

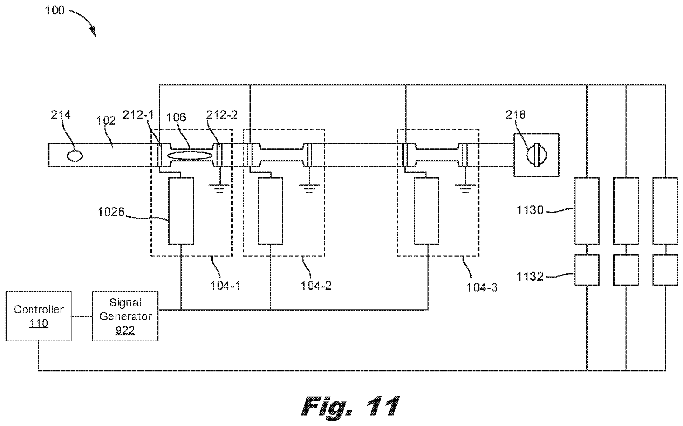

Fig. 11 is a diagram of a cell preparation system (100) with a series of detection devices (104), according to an example of the principles described herein. Fig. 11 depicts various components of the cell preparation system (100) including the fluidic channel (102), detection devices (104-1, 104-2, 104-3), constriction (106), electrode (212), controller (110), and ejector (218) along with the cell (214) that is passed through the fluidic channel (102).

Fig. 11 also depicts the signal generator (922) which as described above may generate multiple signals which are passed to filter devices (1028) of each detection device (104) such that each detection device (104) receives a subset of the signals generated.

Fig. 11 also depicts the sense circuitry (924). In the example depicted in Fig. 11, the isolation component (1130) may be a band-pass filter to gate signals to a particular frequency range. As described above, the sense circuitry (924) also includes a current meter (1132) to measure the current sensed by the electrodes (212). This sensed current is passed to the controller (110) which, along with knowing the input voltage, can determine the impedance in each respective detection device (104).

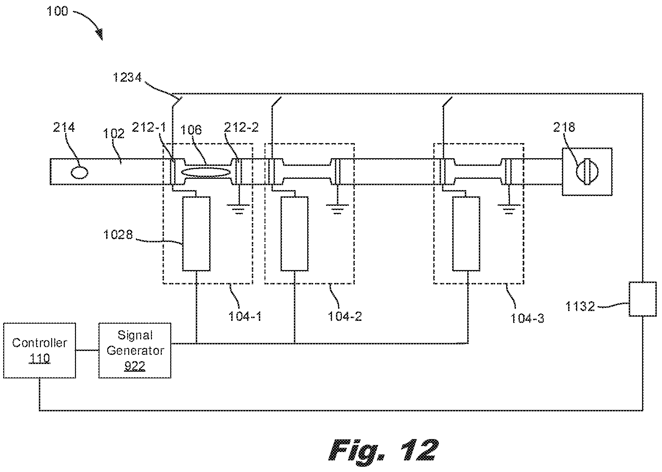

Fig. 12 is a diagram of a cell preparation system (100) with a series of detection devices (104), according to an example of the principles described herein. Fig. 12 depicts various components of the cell preparation system (100) including the fluidic channel (102), detection devices (104-1, 104-2, 104-3), constriction (106), electrode (212), controller (110), signal generator (922), and ejector (218) along with the cell (214) that is passed through the fluidic channel (102).

Fig. 12 also depicts the sense circuitry (924). In the example depicted in Fig. 12, the isolation component may be a switch (1234), that via activation by a transistor control line, selectively couples an output of a detection device (104) to the current meter (1132).

Fig. 13 is a diagram of a cell preparation system (100) with a series of detection devices (104), according to an example of the principles described herein. As described above, the cell preparation system (100) may include one or multiple fluid channels (102-1, 102-2). Through each fluid channel (102-1, 102-2), an individual cell (214-1, 214-2) may pass one at a time for analysis. That is, the cell preparation system (100) includes a sequence of detection devices (Fig. 1, 104) that are parallel to one another. In this example, a sample is passed to each fluid channel (102-1, 102-2) via a fluid feed slot (1336). For simplicity in Fig. 13, reference numbers for some previously described components are omitted. The multiple parallel series of detection devices (Fig. 1, 104) facilitate the processing of more cells (Fig. 2, 214). That is, cells (214) are passed to each series in parallel even though each series may analyze a single cell (Fig. 2, 214) at a time.

In summary, using such a cell preparation system 1) provides highly accurate cell separation and identification; 2) is low cost; 3) provides for the rapid generation of many singulated cells; 4) avoids additional labelling reagents; 5) provides an integrated design; 6) exhibits a low probability of empty wells; and 7) reduces quality control measures. However, the devices disclosed herein may address other matters and deficiencies in a number of technical areas.

## Claims

1. A method (300, 400) of cell preparation, comprising:

    applying (301, 401) different electrical fields to each of a series of detection devices along a fluidic channel (102) of a system, each detection device comprising a constriction configured to deform a cell found therein and at least one electrode pair (212) of a sensor (108) configured to measure an electrical property within the constriction;
    the method further comprising,

        for each detection device:

            introducing (302, 402) a cell into the constriction of the detection device; and
            determining (303, 403), with the at least one electrode pair (212) of the sensor (108), an electrical impedance when the cell is in the constriction; and

        based on a comparison of electrical impedance measures from each detection device:

            verifying (304, 404) a single file transport of cells through the fluidic channel past the series of the detection devices; and
            identifying (305, 405) cells that pass through the fluidic channel.

2. The method of claim 1, further comprising, based on electrical impedance measures from each detection device, determining (406) whether to eject the cell onto a target surface or to dispose of the cell to a waste receptacle.

3. The method of claim 1, further comprising recording (407) properties for each cell.

4. A cell preparation system comprising:

    a fluidic channel (102) configured to transport cells (214) in single file past a series of detection devices (104);
    each detection device comprising:

        a constriction (106) configured to deform a cell found therein; and
        a sensor (108) configured to measure a state within the constriction, whereby each sensor comprises at least one electrode pair (212) configured to measure an impedance within the constriction;

a signal generator (922) configured to generate electrical fields to apply to the series of detection devices;

sense circuitry (924) configured to determine the impedance within each detection device;

an ejector (218) configured to eject cells from the fluidic channel; and

a controller (110) configured to operate the system according to the method of claim 1.

5. The system of claim 4, wherein the controller is configured to identify, per detection device and based on output from the sensors, a cell elasticity, a cell size, and a cell effective conductivity and permittivity.

6. The system of claim 4, wherein the sensor is disposed within the constriction.

7. The system of claim 4, wherein the sensor is disposed outside the constriction.

8. The system of claim 4, wherein a constriction shape varies for at least one detection device.

9. The system of claim 4, further comprising a database (1026), wherein the database:

Is configured to map, per detection device, sensor outputs to quantity and types of cells; and is indexed based on properties of a respective detection device.

10. The system of claim 4, whereby:
the controller is configured to aggregate outputs from multiple electrode pairs to determine whether to eject the cell onto a target surface or to dispose of the cell to a waste receptacle.

11. The system of claim 10, further comprising a filter device (1028) per detection device configured to generate an electrical field specific to a respective detection device.

12. The system of claim 11, wherein the filter device is selected from the group consisting of a band-pass filter and a lock-in amplifier.

13. The system of claim 10, wherein the sense circuitry comprises:

an isolation component (1130) configured to isolate output from a particular detection device, wherein the isolation component is selected from the group consisting of:

a band-pass filter; and

a switch (1234); and

a current meter (1132).

14. The system of any of claims 4 to 13, whereby a variety of constrictions (106) have different shapes or sizes.

**Patentansprüche**

1. Verfahren (300, 400) einer Zellpräparation, das umfasst:

Anlegen (301, 401) unterschiedlicher elektrischer Felder an jede einer Reihe von Detektionsvorrichtungen entlang eines fluidischen Kanals (102) eines Systems, wobei jede Detektionsvorrichtung eine Verengung, die dazu konfiguriert ist, eine darin befindliche Zelle zu verformen, und mindestens ein Elektrodenpaar (212) eines Sensors (108) umfasst, das dazu konfiguriert ist, eine elektrische Eigenschaft innerhalb der Verengung zu messen;
wobei das Verfahren ferner umfasst,
für jede Detektionsvorrichtung:

Einführen (302, 402) einer Zelle in die Verengung der Detektionsvorrichtung; und
Bestimmen (303, 403), mit dem mindestens einen Elektrodenpaar (212) des Sensors (108), einer elektrischen Impedanz, wenn die Zelle in der Verengung ist; und
auf der Basis von einem Vergleich von elektrischen Impedanzmesswerten von jeder Detektionsvorrichtung:

Verifizieren (304, 404) eines Transports von Zellen hintereinander durch den Fluidkanal hindurch an der Reihe der Detektionsvorrichtungen vorbei; und
Identifizieren (305, 405) von Zellen, die durch den Fluidkanal hindurchgehen.

2. Verfahren nach Anspruch 1, das ferner, auf der Basis von elektrischen Impedanzmessungen von jeder Detektionsvorrichtung, ein Bestimmen (406) umfasst, ob die Zelle auf eine Zieloberfläche ausgestoßen werden soll oder die Zelle in einen Abfallbehälter entsorgt werden soll.

3. Verfahren nach Anspruch 1, das ferner ein Aufzeichnen (407) von Eigenschaften für jede Zelle umfasst.

4. Zellpräparationssystem, das umfasst:

einen fluidischen Kanal (102), der dazu konfiguriert ist, Zellen (214) hintereinander an einer Reihe von Detektionsvorrichtungen (104) vorbei zu transportieren;

wobei jede Detektionsvorrichtung umfasst:

eine Verengung (106), die dazu konfiguriert ist, eine darin befindliche Zelle zu verformen; und

einen Sensor (108), der dazu konfiguriert ist, einen Zustand innerhalb der Verengung zu messen, wobei jeder Sensor mindestens ein Elektrodenpaar (212) umfasst, das dazu konfiguriert ist, eine Impedanz innerhalb der Verengung zu messen;

einen Signalgenerator (922), der dazu konfiguriert ist, elektrische Felder zum Anlegen an die Reihe von Detektionsvorrichtungen zu generieren;

eine Erfassungsschaltung (924), die dazu konfiguriert ist, die Impedanz innerhalb jeder Detektionsvorrichtung zu bestimmen;

eine Ausstoßeinrichtung (218), die dazu konfiguriert ist, Zellen aus dem fluidischen Kanal auszustoßen; und

eine Steuereinrichtung (110), die dazu konfiguriert ist, das System nach dem Verfahren nach Anspruch 1 zu betreiben.

5. System nach Anspruch 4, wobei die Steuereinrichtung dazu konfiguriert ist, pro Detektionsvorrichtung und auf der Basis von einer Ausgabe von den Sensoren, eine Zellelastizität, eine Zellgröße und eine effektive Leitfähigkeit und Permittivität einer Zelle zu identifizieren.

6. System nach Anspruch 4, wobei der Sensor innerhalb der Verengung angeordnet ist.

7. System nach Anspruch 4, wobei der Sensor außerhalb der Verengung angeordnet ist.

8. System nach Anspruch 4, wobei eine Verengungsform für mindestens eine Detektionsvorrichtung variiert.

9. System nach Anspruch 4, das ferner eine Datenbank (1026) umfasst, wobei die Datenbank:

dazu konfiguriert ist, pro Detektionsvorrichtung, Sensorausgaben einer Menge und Arten von Zellen zuzuordnen; und
auf der Basis von Eigenschaften einer jeweiligen Detektionsvorrichtung indiziert wird.

10. System nach Anspruch 4, wobei:
die Steuereinrichtung dazu konfiguriert ist, Ausgaben von mehreren Elektrodenpaaren dazu zu aggregieren, zu bestimmen, ob die Zelle auf eine Zieloberfläche ausgestoßen werden soll oder die Zelle in einen Abfallbehälter entsorgt werden soll.

11. System nach Anspruch 10, das ferner eine Filtervorrichtung (1028) pro Detektionsvorrichtung umfasst, die dazu konfiguriert ist, ein für eine jeweilige Detektionsvorrichtung spezifisches elektrisches Feld zu generieren.

12. System nach Anspruch 11, wobei die Filtervorrichtung aus der Gruppe ausgewählt ist, bestehend aus einem Bandpassfilter und einem Lock-in-Verstärker.

13. System nach Anspruch 10, wobei die Erfassungsschaltung umfasst:
eine Isolationskomponente (1130), die dazu konfiguriert ist, eine Ausgabe von einer speziellen Detektionsvorrichtung zu isolieren, wobei die Isolationskomponente aus der Gruppe ausgewählt ist, bestehend aus den Folgenden:

einem Bandpassfilter; und
einem Schalter (1234); und
einem Strommesser (1132).

14. System nach einem der Ansprüche 4 bis 13, wobei eine Vielfalt von Verengungen (106) unterschiedliche Formen oder Größen aufweisen.

## Revendications

1. Procédé (300, 400) de préparation de cellules, comprenant :

l'application (301, 401) de différents champs électriques à chacun d'une série de dispositifs de détection le long d'un canal fluidique (102) d'un système, chaque dispositif de détection comprenant un étranglement conçu pour déformer une cellule trouvée dans celui-ci, et au moins une paire d'électrodes (212) d'un capteur (108) configuré pour mesurer une propriété électrique au sein de l'étranglement ;
le procédé comprenant en outre,
pour chaque dispositif de détection :

l'introduction (302, 402) d'une cellule dans l'étranglement du dispositif de détection ; et
la détermination (303, 403), avec l'au moins une paire d'électrodes (212) du capteur (108), d'une impédance électrique lorsque la cellule est dans l'étranglement ; et
en fonction d'une comparaison de mesures d'impédance électrique provenant de chaque dispositif de détection :

la vérification (304, 404) d'un transport en file indienne de cellules à travers le canal fluidique au-delà de la série des dispositifs de détection ; et l'identification (305, 405) de cellules qui traversent le canal fluidique.

2. Procédé selon la revendication 1, comprenant en outre, en fonction de mesures d'impédance électrique provenant de chaque dispositif de détection, le fait de déterminer (406) s'il faut éjecter la cellule sur une surface cible ou se débarrasser de la cellule dans un réceptacle à déchets.

3. Procédé selon la revendication 1, comprenant en outre l'enregistrement (407) de propriétés pour chaque cellule.

4. Système de préparation de cellules comprenant :

un canal fluidique (102) conçu pour transporter des cellules (214) en file indienne au-delà d'une série de dispositifs de détection (104) ; chaque dispositif de détection comprenant :

un étranglement (106) conçu pour déformer une cellule trouvée dans celui-ci ; et un capteur (108) configuré pour mesurer un état au sein de l'étranglement, moyennant quoi chaque capteur comprend au moins une paire d'électrodes (212) configurée pour mesurer une impédance au sein de l'étranglement ;

un générateur de signal (922) configuré pour générer des champs électriques à appliquer à la série de dispositifs de détection ; un système de circuits de captage (924) configuré pour déterminer l'impédance au sein de chaque dispositif de détection ; un éjecteur (218) conçu pour éjecter des cellules hors du canal fluidique ; et un dispositif de commande (110) configuré pour faire fonctionner le système selon le procédé de la revendication 1.

5. Système selon la revendication 4, dans lequel le dispositif de commande est configuré pour identifier, par dispositif de détection et en fonction d'une sortie provenant des capteurs, une élasticité de cellule, une taille de cellule et une conductivité et une permittivité effectives de cellule.

6. Système selon la revendication 4, dans lequel le capteur est disposé au sein de l'étranglement.

7. Système selon la revendication 4, dans lequel le capteur est disposé à l'extérieur de l'étranglement.

8. Système selon la revendication 4, dans lequel une forme d'étranglement varie pour au moins un dispositif de détection.

9. Système selon la revendication 4, comprenant en outre une base de données (1026), dans lequel la base de données :

est configurée pour mettre en correspondance, par dispositif de détection, des sorties de capteur à une quantité et à des types de cellules ; et est indexée en fonction de propriétés d'un dispositif de détection respectif.

10. Système selon la revendication 4, moyennant quoi : le dispositif de commande est configuré pour agréger des sorties provenant de multiples paires d'électrodes pour déterminer s'il faut éjecter la cellule sur une surface cible ou se débarrasser de la cellule dans un réceptacle à déchets.

11. Système selon la revendication 10, comprenant en outre un dispositif de filtre (1028) par dispositif de détection configuré pour générer un champ électrique spécifique à un dispositif de détection respectif.

12. Système selon la revendication 11, dans lequel le dispositif de filtre est choisi dans le groupe constitué d'un filtre passe-bande et d'un amplificateur synchrone.

13. Système selon la revendication 10, dans lequel le système de circuits de captage comprend : un composant d'isolement (1130) configuré pour isoler une sortie provenant d'un dispositif de détection particulier, dans lequel le composant d'isolement est choisi dans le groupe constitué de :

un filtre passe-bande ; et un commutateur (1234) ; et un ampèremètre (1132).

14. Système selon l'une quelconque des revendications 4 à 13, moyennant quoi une variété d'étranglements (106) ont des formes ou tailles différentes.

Cell Preparation System
100

Fluidic Channel
102

Detection Device
104

Constriction
106

Sensor
108

Controller
110

*Fig. 1*

*Fig. 2*

300

START

Apply different electrical
fields to each of a number of
detection devices along a
fluidic channel
301

For each detection device,
introduce a cell into the
detection device
302

For each detection device,
determine an electrical
impedance when the cell is
in the detection device
303

Based on electrical
impedance measures from
each detection device, verify
a single file transport of cells
through the system
304

Based on electrical
impedance measures from
each detection device,
identify cells that pass
through the fluidic channel
305

END

*Fig. 3*

400

START

Apply different electrical fields to each of a number of detection devices along a fluidic channel
401

For each detection device, introduce a cell into the detection device
402

For each detection device, determine an electrical impedance when the cell is in the detection device
403

Based on electrical impedance measures from each detection device, verify single file transport of cells through the system
404

Based on electrical impedance measures from each detection device, identify cells that pass through the fluidic channel
405

Based on electrical impedance measures from each detection device, determine whether to eject a cell onto a target surface or to dispose of the cell to a waste receptacle
406

Record properties for each analyzed cell
407

END

*Fig. 4*

**Fig. 5**

**Fig. 6**

Fig. 7

*Fig. 8*

Cell Preparation System
100

Signal Generator
922

Fluidic Channel
102

Sense Circuitry
924

Detection Device
104

Constriction
106

Electrode Pair
212

Ejector
218

Controller
110

*Fig. 9*

Cell Preparation System
100

| Signal Generator 922 | Sense Circuitry 924 |
|---|---|
| Fluidic Channel 102 | Ejector 218 |
| Detection Device 104 | Database 1026 |
| Constriction 106 | Filter Device 1028 |
| Electrode Pair 212 | Controller 110 |

Fig. 10

**Fig. 11**

26

*Fig. 12*

**Fig. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5376878 A
- US 2018016539 A1
- WO 2018071731 A1
- US 2020070167 A1

**Non-patent literature cited in the description**

- **YANG DAHOU et al.** *20th international conference on solid-state sensors, actuators and microsystems & eurosensors of the IEEE*, vol. 133, 16-23